Europäisches Patentamt

**European Patent Office**

Office européen des brevets

(11) Veröffentlichungsnummer: **0 183 028**
**B1**

(12)

# EUROPÄISCHE PATENTSCHRIFT

(45) Veröffentlichungstag der Patentschrift:
**01.03.89**

(51) Int. Cl.⁴: **C 07 C 29/10, C 07 C 31/20**

(21) Anmeldenummer: **85113046.8**

(22) Anmeldetag: **15.10.85**

(54) **Verfahren zur kontinuierlichen Herstellung vicinaler Diole.**

(30) Priorität: **24.11.84 DE 3442938**

(43) Veröffentlichungstag der Anmeldung:
**04.06.86 Patentblatt 86/23**

(45) Bekanntmachung des Hinweises auf die Patenterteilung:
**01.03.89 Patentblatt 89/9**

(84) Benannte Vertragsstaaten:
**AT CH DE FR GB IT LI NL SE**

(56) Entgegenhaltungen:
**EP-A- 0 025 961**
**BE-A- 717 600**

(73) Patentinhaber: **Degussa Aktiengesellschaft,
Weissfrauenstrasse 9, D-6000 Frankfurt am Main 1 (DE)**

(72) Erfinder: **Siegmeier, Rainer, Dr. Dipl.-Chem.,
Egenolffstrasse 4, D-6000 Frankfurt (DE)**
Erfinder: **Prescher, Günter, Dr. Dipl.-Chem.,
Liesingstrasse 2, D-6450 Hanau 9 (DE)**
Erfinder: **Maurer, Helmut, In den Steinäckern 5,
D-6458 Rodenbach (DE)**

## Beschreibung

Die Erfindung betrifft die Herstellung vicinaler Diole, also von Diolen, deren Hydroxylgruppen an benachbarten Kohlenstoffatomen stehen, durch Verseifung der entsprechenden Epoxide.

Vicinale Diole sind vielfach verwendbar, z.B. als Zwischenprodukte bei der Herstellung von Butadien und Isopren, die aus den entsprechenden Diolen durch Dehydratisierung entstehen.

Ausserdem spielen sie eine Rolle als Komponente bei der Polyester- und Polyurethanherstellung, sowie in der kosmetischen und pharmazeutischen Industrie.

Vicinale Diole werden unter anderem durch Verseifung ihrer entsprechenden Epoxide hergestellt. Dabei wird diese Verseifung sowohl durch Zugabe von Säuren (siehe z.B. den Stand der Technik der US-PS 3 576 890), wie Alkalien (siehe z.B. DE-OS 1 793 247, DE-OS 2 203 806), wie Salzen (aliphatischer Mono- oder Polycarbonsäuren (DE-OS 2 256 907), sowie primären, sekundären oder tertiären Aminsalzen oder Ammoniumsalzen (EP-OS 0 025 961) katalysiert.

Es ist auch bekannt, die als Katalysator fungierende Säure durch Zugabe von Estern niederer Carbonsäuren und deren Hydrolyse zu Alkohol und Säuren zu erzeugen (US-PS 3 576 890).

Die Verseifung kann in rein wässrigem Medium oder in Gegenwart von Lösungsvermittlern wie wasserlöslichen Ketonen oder cyclischen Äthern (DE-OS 2 256 907) vorgenommen werden.

Die Verseifung braucht nicht nur direkt mit den Epoxiden zu erfolgen, sondern es ist auch möglich, zunächst z.B. einen niederen Carbonsäureester zu bilden, der dann der Verseifung unterliegt (J. Am. Chem. Soc. 66, Seite 1925, 1944). Dieses Verfahren wird meist nur bei langkettigen Epoxiden angewendet.

Neben der Ausbeute an Diolen ist auch deren Selektivität massgebend. Letztere wird durch einen möglichst konstant gehaltenen pH-Wert, der in schwach saurem Gebiet liegt, beeinflusst. Zu diesem Zweck wurden Verfahren entwickelt, die in Gegenwart von Kohlendioxid arbeiten (siehe US-PS 2 623 909, DE-OS 2 615 595). Hierbei musste diskontinuierlich im Autoklaven unter teilweise erheblichen Drucken gearbeitet werden, siehe die dort zitierten Beispiele.

Nach dem Stand der Technik wurden in erster Linie entweder niedere Epoxide wie Äthylen- oder Propylenoxid oder höhere Epoxide mit z.B. 12 bis 18 C-Atomen zu den entsprechenden vicinalen Diolen verseift.

Wenn bei diesen Verfahren zwar allgemeine Bereiche für die Kohlenstoffzahl der Epoxide angegeben wurden, die bei den niederen Epoxiden bis auf etwa $C_5$ herauf- und bei den höheren Epoxiden bis auf etwa $C_4$ heruntergingen, so wurden in den Beispielen Äthylen- oder Propylenoxid oder aber höhere Epoxide mit z.B. 12 bis 18 C-Atomen verwendet.

Der Zwischenbereich von isomeren Pentenoxiden bis Heptenoxiden und deren direkte Verseifung zu den entsprechenden Diolen – also keine Diolherstellung durch Verseifung der Carbonsäureester der Epoxide – hat bisher noch zu keiner technischen Realisierung geführt. Gerade die Diole dieses Zwischenbereiches sind aber wesentlich bei der Herstellung von z.B. Pestiziden.

Aufgabe der Erfindung ist daher die direkte Verseifung von Epoxiden mit 3 bis 8, bevorzugt 5 bis 7, Kohlenstoffatomen zu den entsprechenden vicinalen Diolen mit guten Ausbeuten und hoher Selektivität.

Es wurde nun gefunden, dass sich vicinale Diole mit 3 bis 8 Kohlenstoffatomen direkt und kontinuierlich durch säurekatalysierte Verseifung mit Wasser in Gegenwart eines organischen Lösungsmittels bei niedrigem Druck und mässigen Temperaturen in hohen Ausbeuten und Selektivitäten herstellen lassen, wenn man eine Lösung aliphatischer linearer oder verzweigter bzw. cyclischer Epoxide mit 3 bis 8 Kohlenstoffatomen in aromatischen oder Halogenkohlenwasserstoffen, die mit Wasser nicht mischbar sind, mit Wasser bei 30 bis 150 °C bei Atmosphärendruck oder einem Druck bis 5 bar in Gegenwart eines sauren Katalysators verseift.

Unter aliphatischen linearen oder verzweigten bzw. cyclischen Epoxiden mit 3 bis 8 C-Atomen werden verstanden: Propylenoxid; 2,3-Epoxypropanol-1; 1,2-Butenoxid; 2,3-Butenoxid; 3,4-Epoxybuten-1; 1,2,3,4-Butadiendioxid; 2,3-Epoxybutan-diol-1,4; 1,2-Epoxy-2-methylbutan; 2-Pentenoxid; Cyclopentenoxid; 2-Methyl-1-butenoxid; 3-Methyl-1-butenoxid; 2-Methyl-2-butenoxid; 2,3-Dimethylbutenoxid-1; 2,3-Dimethylbutenoxid-2; 3,3-Dimethylbutenoxid-1; 1-Hexenoxid; 2-Hexenoxid; 3-Hexenoxid, Cyclohexenoxid; 4-Methylpentenoxid-1; 5,6-Epoxyhexen-1; 1,2,5,6-Diepoxyhexan; 2,3,5,5-Diepoxybicyclo-2,2,1-heptan; 2,3-Epoxydicyclo-2,2,1-heptan; 1-Heptenoxid; 3-Heptenoxid; Cycloheptenoxid; 1-Octenoxid 7,8-Epoxy-octen-1; 1,2,5,6-Diepoxycyclooctan; 1,2,3,4-Diepoxycyclooctan; 1,2-Epoxy-cycloocten-3; 3,4-Epoxy-cyclooctanol; 2,3-Epoxy-bicyclo-3,3,0-octan; Vinylcyclohexenoxid; Vinylcyclohexendioxid.

Als sehr geeignete Ausgangsprodukte erwiesen sich 2-Pentenoxid, 1-Hexenoxid, 1-Neohexenoxid, Cyclohexenoxid, also Epoxide mit 5 bis 7 C-Atomen.

Die Epoxide, die nach den bekannten Verfahren hergestellt werden, fallen im allgemeinen in Form einer Lösung, vor allem in aromatischen oder Halogenkohlenwasserstoffen an (siehe DE-AS 25 19 298, DE-OS 26 02 776). Diese Lösungen können direkt im erfindungsgemässen Verseifungsverfahren eingesetzt werden. Es ist aber auch möglich, ein handelsübliches Epoxid in einem Lösungsmittel zu lösen und diese Lösung dann gemäss der Erfindung zu verseifen. Meist ist hierzu keine zusätzliche Reinigung des Handelsproduktes erforderlich.

Unter aromatischen Kohlenwasserstoffen werden Benzol, Toluol; o-, m-, p-Xylol; Äthylbenzol, Propylbenzol, Pseudocumol, Mesitylen verstanden. Besonders günstig als Lösungsmittel ist Benzol.

Von den halogenierten Kohlenwasserstoffen zeichneten sich besonders die chlorierten Kohlenwasserstoffe als Lösungsmittel aus, z.B. Chloroform, Propylendichlorid, Dichlormethan, die Di- und Trichloräthane. Geeignet war vor allen Dingen Chloroform.

Die Lösungsmittel werden in solchen Mengen eingesetzt, dass sich das verwendete Epoxid homogen löst. Die Epoxide liegen bevorzugt in Lösungen vor, die 15 bis 30 Gewichtsprozent Epoxid enthalten, unabhängig von der Art der Lösungsmittel. Es können aber auch sowohl höher als auch niedriger konzentrierte Lösungen verwendet werden. Das Gewichtsverhältnis von Epoxid zu Wasser liegt bei 1:1 bis 1:5, bevorzugt bei 1:1,5 bis 1:2,5.

Als saure Katalysatoren erwiesen sich die Mineralsäuren, wie Schwefelsäure, Salzsäure, Orthophosphorsäure, Perchlorsäure, als geeignet. Bevorzugt ist Schwefelsäure.

Auch organische Säuren kommen als Katalysatoren infrage, wie Ameisen-, Essig- und Propionsäure, Toluolsulfonsäure, Isobuttersäure, Methansulfonsäure. Günstig war Ameisensäure.

Die Katalysatoren, einerlei, ob als Mineralsäure oder organische Säure, werden in Mengen von 0,05 bis 5 Gewichtsprozent, bezogen auf die Menge Wasser, verwendet.

Bevorzugte Verseifungstemperatur ist der Bereich von 30 bis 120 °C. Die Verseifung findet in üblichen Destillationskolonnen statt, die man bevorzugt bei Atmosphärendruck oder bei leichtem Überdruck bis 5 bar betreibt.

Die Lösung der Epoxide wird in die Mitte der Verseifungskolonne eingeführt und das Wasser, das den sauren Katalysator enthält, nahe dem Kopf der Kolonne eingegeben.

Bei der Destillation wird am Kopf der Kolonne das Lösungsmittel, eventuell in Form eines Azeotrops mit Wasser, abgenommen.

Es erwies sich, dass ein Verhältnis «Lösungsmittel zu Wasser» von 5:1 bis 1:1 Gewichtsteilen recht günstig war. Sehr geeignet war ein Verhältnis von 1,5:1 bis 2:1 Gewichtsteil.

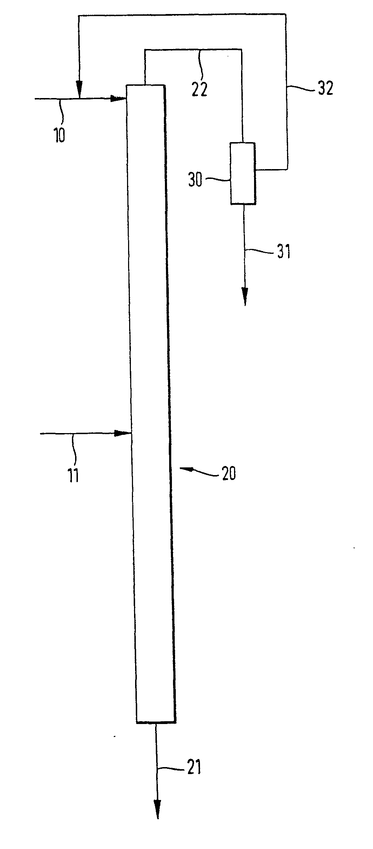

Das erfindungsgemässe Verfahren wird anhand der Abbildung 1 näher erläutert, bei der über Kopf ein Azeotrop mit Wasser abgenommen wird.

Die Lösung des Epoxids wird über Leitung 11 in die Mitte der Verseifungskolonne 20 eingeführt. Am Kopf der Kolonne wird Frischwasser über Leitung 10, vermischt mit rückgeführtem Wasser (über Leitung 32), eingegeben. Das über Leitung 10 einströmende Wasser enthält den sauren Katalysator. Am Kopf geht über Leitung 22 das Azeotrop aus Lösungsmittel und Wasser ab, das im Wasserscheider 30 in Wasser und Lösungsmittel getrennt wird. Das Lösungsmittel verlässt über Leitung 31 die Kolonne, das Wasser wird über Leitung 32 in Leitung 10 rückgeführt.

Im Destillationssumpf der Kolonne 20 wird eine saure wässrige Lösung des Epoxids in Wasser erhalten, die über Leitung 21 abgezogen wird.

Der technische Fortschritt des erfindungsgemässen Verfahrens liegt in der Möglichkeit, die Verseifung der Epoxide direkt und kontinuierlich ohne Anwendung hoher Temperaturen oder Drucke durchzuführen und dabei hohe Ausbeuten und hohe Selektivitäten zu erhalten. Auch spezielle Zusätze zur Beeinflussung der Selektivität sind nicht nötig. Ein technisch so einfaches Verfahren zur kontinuierlichen Herstellung vicinaler Diole aus den entsprechenden Epoxiden war bisher nicht bekannt.

Das Verfahren wird in den folgenden Beispielen näher erläutert. In diesen Beispielen werden die Ergebnisse, die diskontinuierlich erhalten wurden, mit denen nach dem kontinuierlichen erfindungsgemässen Prozess verglichen.

Die diskontinuierlichen Versuche wurden einmal in Anwesenheit von Lösungsmitteln (Beispiele 7 bis 12) und zum anderen ohne Lösungsmittel durchgeführt (Beispiele 1 bis 6). Die kontinuierlichen erfindungsgemässen Beispiele in Gegenwart von Lösungsmittel (Benzol) tragen die Ziffern 11 bis 18.

Eingesetzt wurden Propylenoxid, 2-Pentenoxid, 1-Neohexenoxid, (3,3-Dimethyl-1,2-epoxybutan), 1-Hexenoxid und Cyclohexenoxid sowie 1-Octenoxid.

Die diskontinuierlichen Versuche 1 bis 12 wurden folgendermassen durchgeführt:

In einem Dreihalskolben mit Rührer, Innenthermometer und Rückflusskühler wurden 100 g Epoxid, 200 g Wasser und gegebenenfalls 400 g Benzol vorgelegt, mit 0,1 Gewichtsprozent Schwefelsäure versetzt und zum Sieden erhitzt. Nach Reaktionsende wurden Epoxid-Umsatz, Ausbeute und die Menge der gebildeten hochsiedenden Nebenprodukte titrimetrisch und gaschromatographisch ermittelt.

Für die kontinuierlichen Beispiele 13 bis 16 finden sich die Einsatzmengen in Tabelle I. Die benzolische Lösung des entsprechenden Epoxids wird gemäss Abbildung 1 über Leitung 11 in die Verseifungskolonne 20 eingeführt. Gleichzeitig werden über die Zuleitung 10 die in Tabelle I angegebenen Wassermengen mit den dort angegebenen Schwefelsäuremengen eingespeist. Das über Kopf der Kolonne abgehende Benzol-Wasser-Azeotrop wird in dem Wasserscheider 30 in Benzol, das über Leitung 32 in Leitung 10 rückgeführt wird, und Wasser getrennt.

Über Leitung 21 wird das vicinale Diol in wässrig saurer Lösung abgenommen. Die Ergebnisse der diskontinuierlichen Beispiele mit und ohne Lösungsmittel, sowie der erfindungsgemässen kontinuierlichen Beispiele sind in Tabelle II zusammengestellt.

Als Verseifungszeit für die kontinuierlichen Beispiele wurden 1,4 bis 1,6 Stunden, abhängig vom Durchsatz, ermittelt.

Aus Tabelle II geht deutlich die Ausbeutesteigerung nach der kontinuierlichen Methode in Gegenwart des Lösungsmittels sowie die wesentliche Erhöhung der Selektivität (Menge der hochsiedenden Nebenprodukte) hervor gegenüber den diskontinuierlichen Verfahren. Während die Anwesenheit des Lösungsmittels im diskontinuierlichen Verfahren eine gewisse Verbesserung der Selektivität, aber keine allgemeine Verbesserung der Ausbeute hervorruft (Beispiele 7 bis 12 im Vergleich zu Beispielen 1 bis 6), wird im erfindungsgemässen Verfahren sowohl Ausbeute als auch Selektivität erheblich verbessert (Beispiele 13 bis 18).

Tabelle I

| Epoxid | Einsatz/h g | Wasser- menge/h/g | konz. $H_2SO_4$ Gew.-% | Produkt |
|---|---|---|---|---|
| Propylenoxid | 102 | 212 | 1 | Propandiol-1,2 |
| Pentenoxid-2 | 95 | 185 | 0,1 | Pentandiol-2,3 |
| Hexenoxid-1 | 107 | 212 | 0,1 | Hexandiol-1,2 |
| Neohexenoxid-1 | 106 | 210 | 1 | Neohexandiol-1,2 |
| Cyclohexenoxid | 111 | 216 | 0,1 | Cyclohexandiol-1,2 |
| Octenoxid-1 | 106 | 212 | 0,3 | Octandiol-1,2 |

Die Epoxide wurden jeweils als ca. 20-gew.-%ige Lösung in Benzol eingesetzt.

Tabelle II

| Nr. | Epoxid | Lösungs- mittel | konz. $H_2SO_4$ Gew.-% | Reaktions- zeit h | Ausbeute % | hochs. Neben- produkte g/100 g Diol |
|---|---|---|---|---|---|---|
| 1 | Propylenoxid | — | 0,1 | 0,6 | 77 | 18,2 |
| 2 | Pentenoxid-2 | — | 0,1 | 0,3 | 87 | 7,4 |
| 3 | Hexenoxid-1 | — | 0,1 | 0,3 | 88 | 10,5 |
| 4 | Cyclohexenoxid | — | 0,1 | 0,2 | 94 | 3,5 |
| 5 | Neohexenoxid-1 | — | 1,0 | 0,3 | 86 | 10,4 |
| 6 | Octenoxid-1 | — | 0,1 | 1,5 | 76 | 19,6 |
| | | diskontinuierlich, mit Lösungsmittel | | | | |
| 7 | Propylenxoid | Benzol | 0,1 | 1,2 | 77 | 15,6 |
| 8 | Pentenoxid-2 | Benzol | 0,1 | 8 | 87 | 6,0 |
| 9 | Hexenoxid-1 | Benzol | 0,4 | 7 | 84 | 11,2 |
| 10 | Cyclohexenoxid-1 | Benzol | 0,1 | 0,5 | 96 | 1,2 |
| 11 | Neohexenoxid-1 | Benzol | 3,0 | 4,2 | 79 | 14,2 |
| 12 | Octenoxid-1 | Benzol | 3,0 | 4 | 64 | 26,0 |
| | | erfindungsgemäßes, kontinuierliches Verfahren | | | | |
| 13 | Propylenoxid | Benzol | 1,0 | — | 95 | 4,2 |
| 14 | Pentenoxid-2 | Benzol | 0,1 | — | 99 | 0,8 |
| 15 | Hexenoxid-1 | Benzol | 0,1 | — | 99 | 0,8 |
| 16 | Cyclohexenoxid | Benzol | 0,1 | — | 99 | 0,3 |
| 17 | Neohexenoxid-1 | Benzol | 1,0 | — | 99 | 0,2 |
| 18 | Octenoxid-1 | Benzol | 0,3 | — | 89 | 8,1 |

**Patentansprüche**

1. Kontinuierliches Verfahren zur Herstellung niedermolekularer vicinaler Diole durch säurekatalysierter Verseifung der entsprechenden Epoxide mit Wasser in Gegenwart eines organischen Lösungsmittels bei niedrigem Druck und mässigen Temperaturen, dadurch gekennzeichnet, dass man eine Lösung aliphatischer linearer oder verzweigter bzw. cyclischer Epoxide mit 3 bis 8 Kohlenstoffatomen in aromatischen oder Halogenkohlenwasserstoffen, die mit Wasser nicht mischbar sind, mit Wasser bei 30 bis 150 °C bei Atmosphärendruck oder einem Druck bis 5 bar in Gegenwart eines sauren Katalysators verseift.

2. Verfahren nach Anspruch 1, dadurch gekennzeichnet, dass man Benzol oder Chloroform als organisches Lösungsmittel verwendet.

3. Verfahren nach den Ansprüchen 1 und 2, dadurch gekennzeichnet, dass man 1-Hexen-, 1-Neohexenoxid oder Cyclohexenoxid als Epoxid einsetzt.

4. Verfahren nach den Ansprüchen 1 bis 3, dadurch gekennzeichnet, dass man Epoxid und Wasser im Gewichtsverhältnis von 1:1 bis 1:5, bevorzugt von 1:1,5 bis 1:2,5 einsetzt.

5. Verfahren nach den Ansprüchen 1 bis 4, dadurch gekennzeichnet, dass man ein Verhältnis von Lösungsmittel zu Wasser von 5:1 bis 1:1 Gewichtsteilen, bevorzugt von 1,5:1 bis 2:1 Gewichtsteilen, verwendet.

6. Verfahren nach den Ansprüchen 1 bis 5, dadurch gekennzeichnet, dass man den sauren Katalysator in Mengen von 0,05 bis 5 Gewichtsprozent, bezogen auf die Menge Wasser, verwendet.

7. Verfahren nach den Ansprüchen 1 bis 6, dadurch gekennzeichnet, dass man als Katalysator Mineralsäuren, wie Schwefelsäure, Salzsäure, Orthophosphorsäure, Perchlorsäure, einsetzt.

8. Verfahren nach den Ansprüchen 1 bis 6, dadurch gekennzeichnet, dass man als Katalysator organische Säuren, wie Ameisensäure, Essigsäure, Propionsäure oder Toluolsulfonsäure, Methansulfonsäure, Isobuttersäure, verwendet.

## Revendications

1. Procédé continu pour la production de diols vicinaux à bas poids moléculaires, par saponification, catalysée par un acide, des époxydes correspondants, avec de l'eau en présence d'un solvant organique, à basse pression et moyennes températures, caractérisé en ce que l'on saponifie une solution d'époxydes aliphatiques linéaires ou ramifiés ou cycliques ayant de 3 à 8 atomes de carbone, dans des hydrocarbures aromatiques ou halogénés qui ne sont pas miscibles à l'eau, avec de l'eau, à 30–150 °C, sous la pression atmosphérique ou sous une pression allant jusqu'à 5 bars, en présence d'un catalyseur acide.

2. Procédé selon la revendication 1, caractérisé en ce que l'on utilise en tant que solvant organique le benzène ou le chloroforme.

3. Procédé selon les revendications 1 et 2, caractérisé en ce que l'on utilise en tant qu'époxyde l'oxyde d'hexène-1, l'oxyde de néohexène-1 ou l'oxyde de cyclohexène.

4. Procédé selon l'une quelconque des revendications 1 à 3, caractérisé en ce que l'on utilise l'époxyde et l'eau dans le rapport pondéral de 1:1 à 1:5, de préférence de 1:1,5 à 1:2,5.

5. Procédé selon l'une quelconque des revendications 1 à 4, caractérisé en ce que l'on utilise un rapport du solvant à l'eau allant de 5:1 à 1:1 parties en poids, de préférence de 1,5:1 à 2:1 parties en poids.

6. Procédé selon l'une quelconque des revendications 1 à 5, caractérisé en ce que l'on utilise le catalyseur acide en quantités de 0,05 à 5% en poids, par rapport à la quantité d'eau.

7. Procédé selon l'une quelconque des revendications 1 à 6, caractérisé en ce que l'on utilise en tant que catalyseur des acides minéraux, tels que l'acide sulfurique, l'acide chlorhydrique, l'acide orthophosphorique, l'acide perchlorique.

8. Procédé selon l'une quelconque des revendications 1 à 6, caractérisé en ce que l'on utilise en tant que catalyseur des acides organiques, tels que l'acide formique, l'acide acétique, l'acide propionique ou l'acide toluènesulfonique, l'acide méthanesulfonique, l'acide isobutyrique.

## Claims

1. Continuous process for the preparation of low-molecular-weight vicinol diols by acid-catalysed hydrolysis of the corresponding epoxides with water in the presence of an organic solvent at low pressure and moderate temperatures, characterised in that a solution of aliphatical linear or branched or cyclic epoxides having 3 to 8 carbon atoms in aromatic or halogenated hydrocarbons which are water-immiscible is hydrolysed with water at 30 to 150 °C at atmospheric pressure or a pressure up to 5 bar in the presence of an acid catalyst.

2. Process according to Claim 1, characterised in that the organic solvent used is benzene or chloroform.

3. Process according to Claims 1 and 2, characterized in that 1-hexene oxide, 1-neohexene oxide or cyclohexene oxide is used as the epoxide.

4. Process according to Claims 1 to 3, characterized in that the epoxide and water are used in a weight ratio of 1:1 to 1:5, preferably of 1:1.5 to 1:2.5.

5. Process according to Claims 1 to 4, characterized in that a ratio of solvent to water of 5:1 to 1:1 of parts by weight, preferably 1.5:1 to 2:1 part by weight, is used.

6. Process according to Claims 1 to 5, characterized in that the acid catalyst is used in amounts vo 0.05 to 5% by weight, relative to the amount of water.

7. Process according to Claims 1 to 6, characterized in that the catalyst used consists of mineral acids such as sulphuric acid, hydrochloric acid, orthophosphoric acid, and perchloric acid.

8. Process according to Claims 1 to 6, characterized in that the catalyst used consists of organicacids such as formic acid, acetic acid, propionic acid or toluenesulphonic acid, methanesulphonic acid or isobutyric acid.